(19) Europäisches Patentamt
European Patent Office
Office européen des brevets

(11) Veröffentlichungsnummer: **0 286 896 B1**

(12) # EUROPÄISCHE PATENTSCHRIFT

(45) Veröffentlichungstag der Patentschrift: **02.09.92**

(51) Int. Cl.[5]: **C07C 211/53**, A61K 7/13

(21) Anmeldenummer: **88104956.3**

(22) Anmeldetag: **28.03.88**

(54) **Aminophenylalkylendiamine und deren Verwendung in Oxidationshaarfärbemitteln.**

(30) Priorität: **06.04.87 DE 3711579**

(43) Veröffentlichungstag der Anmeldung:
**19.10.88 Patentblatt 88/42**

(45) Bekanntmachung des Hinweises auf die Patenterteilung:
**02.09.92 Patentblatt 92/36**

(84) Benannte Vertragsstaaten:
**AT BE CH DE ES FR GB IT LI NL SE**

(56) Entgegenhaltungen:
**DE-A- 1 939 062**
**GB-A- 2 018 302**
**GB-A- 2 018 836**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Rose, David, Dr.**
**Holbeinweg 7**
**W-4010 Hilden(DE)**
Erfinder: **Lieske, Edgar**
**Hunsrückenstrasse 40**
**W-4000 Düsseldorf(DE)**
Erfinder: **Maak, Norbert, Dr.**
**Im Jagdfeld 41 a**
**W-4040 Neuss(DE)**

**EP 0 286 896 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue Aminophenylalkylendiamine und deren Salze sowie deren Verwendung als Entwicklerkomponente in Oxidationshaarfärbemitteln.

Für das Färben von Haaren spielen die sogenannten Oxidationshaarfärbemittel wegen ihrer intensiven Farben und guten Echtheitseigenschaften eine bevorzugte Rolle. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf menschlichem Haar besitzen, wobei keine merklichen Unterschiede zwischen strapaziertem und frisch nachgewachsenem Haar bestehen dürfen. Sie sollen beständig sein gegen Licht, Wärme und den Einfluß chemischer Reduktionsmittel, z. B. gegen Dauerwellflüssigkeiten. Schließlich sollen sie die Kopfhaut nicht zu sehr anfärben und vor allem sollen sie in toxikologischer und dermatologischer Hinsicht unbedenklich sein.

Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate und 4-Aminopyrazolonderivate eingesetzt. Als sogenannte Kupplersubstanzen werden m-Phenylendiaminderivate, Naphthole, Resorcinderivate und Pyrazolone verwendet.

Eine besondere Bedeutung kommt der Intensität der bei der oxidativen Kupplung gebildeten Farbnuancen und den Echtheitseigenschaften, insbesondere der Lichtechtheit zu. Aus DE-OS 19 39 062 waren bereits N,N'-Bis-(4-aminophenyl)-ethylendiamine als Oxidationsfarbstoffvorprodukte in Haarfärbemitteln bekannt. Die mit diesen Entwicklerkomponenten mit üblichen Kupplerverbindungen erhältlichen Haaranfärbungen genügen nicht den hohen Anforderungen an Intensität und Echtheitseigenschaften.

Es wurde nun gefunden, daß wesentlich intensivere Haaranfärbungen erreicht werden können, wenn als Entwicklerkomponente N-4-Aminophenyl-N'-2,4-diaminophenyl-alkylendiamine eingesetzt werden.

Gegenstand der Erfindung sind N-4-Aminophenyl-N'-2,4-diaminophenyl-alkylendiamine der Formel (I)

(I) 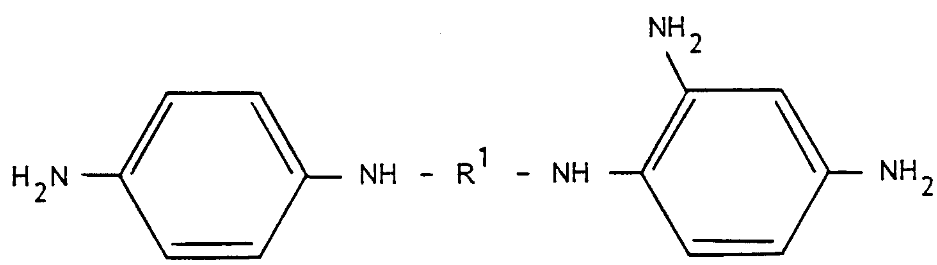

in der $R^1$ eine Gruppe $-C_nH_{2n}-$, worin n = 2 bis 4 ist oder eine Gruppe

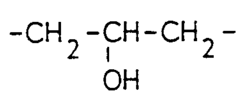

darstellt und deren wasserlösliche Salze. Von den Verbindungen dieser Formel kommt dem N-4-Aminophenyl-N'-2,4-diaminophenyl-ethylendiamin die größte Bedeutung zu.

Die Verbindungen der Formel (I) sind neu. Sie lassen sich nach einem an sich üblichen Verfahren dadurch herstellen, daß man N-(p-Nitrophenyl)-alkylendiamine der Formel (II)

(II) 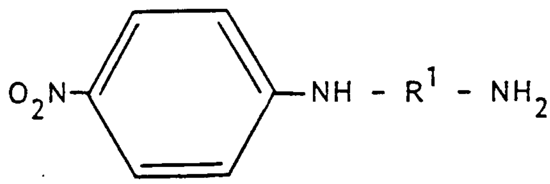

mit 2,4-Dinitrofluorbenzol umsetzt und das dabei erhaltene N-4-Nitrophenyl-N'-2,4-dinitrophenyl-alkylendiamin katalytisch zum N-4-Aminophenyl-N'-2,4-diaminophenylalkylendiamin der Formel (I) hydriert.

Die neuen Verbindungen der Formel (I) oder deren Salze eignen sich zur Verwendung als Oxidationsfarbstoffvorprodukte vom Entwicklertyp in Haarfärbemitteln. Sie vermögen unter der Einwirkung von Oxidationsmitteln Farbstoffe auszubilden. Besonders intensive und brillante Farbstoffe werden jedoch durch oxidative Kupplung in Gegenwart von Kupplersubstanzen gebildet.

Ein weiterer Erfindungsgegenstand sind Haarfärbemittel mit einem Gehalt an Oxidationsfarbstoffvorprodukten in einem kosmetischen Träger, die als Oxidationsfarbstoffvorprodukte N-4-Aminophenyl-N'-2,4-diaminophenyl-alkylendiamine der Formel (I) oder deren wasserlösliche Salze als Entwicklerkomponente neben üblichen Entwickler- oder Kupplerkomponenten und gegebenenfalls direktziehenden Haarfarbstoffen enthalten. Unter den zahlreichen bekannten Kupplersubstanzen sind für die neuen Entwickler der Formel (I) besonders solche mit phenolischen Hydroxylgruppen und solche vom Typ der Bis-(2,4-diaminophenyl)-alkane und der Bis-(2,4-diaminophenoxy)-alkane geeignet, wie sie in DE-OS -32 35 615 und DE-OS 28 52 272 beschrieben sind. Eine weitere, besonders bevorzugte Ausführungsform der Erfindung sind daher Haarfärbemittel der obengenannten Art, die als Kupplerkomponente Phenole, Naphthole, Resorcine und/oder 1,3-Bis-(2,4-diaminophenyl)-alkane oder 1,3-Bis-(2,4-diaminophenoxy)-alkane enthalten. Solche Haarfärbemittel liefern besonders intensive Färbungen im Bereich blauroter, violetter, blauer und schwarzer Nuancen.

Die erfindungsgemäßen N-4-Aminophenyl-N'-2,4-diaminophenyl-alkylendiamine der Formel (I) können entweder als solche oder in Form ihrer wasserlöslichen Salze mit anorganischen oder organischen Säuren, z. B. als Hydrochloride, Sulfate, Phophate, Acetate, Propionate, Lactate oder Citrate isoliert und in Haarfärbemitteln eingesetzt werden.

In den erfindungsgemäßen Haarfärbemitteln werden die Entwicklersubstanzen und die Kupplersubstanzen im allgemeinen in äquimolaren Mengen eingesetzt, ein gewisser Überschuß einzelner Oxidationsfarbstoffvorprodukte ist jedoch nicht nachteilig, so daß Entwicklersubstanzen und Kupplersubstanzen in einem Molverhältnis von 1 : 0,5 bis 1 : 2 eingesetzt werden können. Die erfindungsgemäßen N-4-Aminophenyl-N'-2,4-diaminophenylalkylendiamine der Formel (I) oder deren Salze können in einer Menge von 0,05 bis 10 Millimol pro 100 g des Haarfärbemittels verwendet werden. Es ist dabei nicht erforderlich, daß die Verbindungen der Formel (I) einheitliche Verbindungen sind. Vielmehr können auch Gemische der Verbindungen zum Einsatz kommen.

Zur Modifikation der Haaranfärbung können den erfindungsgemäßen Haarfärbmitteln auch bekannte direktziehende Haarfarbstoffe, z. B. Nitrophenylendiaminderivate, Anthrachinonfarbstoffe oder Indophenole zugesetzt werden.

Zur Herstellung der erfindungsgemäßen Haarfärbemittel werden die Oxidationsfarbstoffvorprodukte und gegebenenfalls direktziehende Farbstoffe in einen geeigneten kosmetischen Träger eingearbeitet. Solche Träger sind z. B. Cremes, Emulsionen, Gele oder auch tensidhaltige, schäumende Lösungen, z. B. Shampoos oder andere Zubereitungen, die für die Anwendung auf dem Haar geeignet sind. Übliche Bestandteile solcher kosmetischer Zubereitungen sind z. B. Netz- und Emulgiermittel wie anionische, nichtionische oder ampholytische Tenside, z. B. Fettalkholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride,Fettsäurealkanolamide sowie Verdickungsmittel wie z. B. Methyl- oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt. Die Oxidationsfarbstoffvorprodukte werden in Mengen von 0,2 bis 5 Gew.-%, vorzugsweise 1 bis 3 Gew.-% des gesamten Färbemittels in den Träger eingemischt.

Eine bevorzugte Anwendungsform für die erfindungsgemäßen N-4-Aminophenyl-N'-2,4-diaminophenyl-

alkylendiamine der Formel (I) sind Cremehaarfärbemittel in Form einer Öl-in-Wasser-Emulsion mit einem Gehalt von

1 bis 10 Millimol pro 100 g an Entwicklerkomponenten,

1 bis 10 Millimol pro 100 g an Kupplerkomponenten,

| | |
|---|---|
| 1 bis 10 Gew.-% | eines Alkyl($C_{10}$-$C_{18}$)-sulfat- oder Alkyl($C_{10}$-$C_{16}$)-ethersulfattensids, |
| 5 bis 20 Gew.-% | eines Fettalkohols oder Fettalkoholgemisches mit 12 bis 18 C-Atomen, |
| 0,1 bis 2 Gew.-% | eines Oxidationsinhibitors, bevorzugt aus der Gruppe Alkalisulfit, Alkaliascorbat oder Alkali-dithionit |

sowie Ammoniak in einer Menge, um den pH-Wert der Emulsion auf einen Wert zwischen 8 und 10 einzustellen.

Das genannte Alkylsulfat- oder Alkylethersulfattensid kann als Alkali-, Ammonium- oder Alkanolammoniumsalz mit 2 oder 3 C-Atomen in der Alkanolgruppe vorliegen, z. B. als Natrium-, Triethanolammonium- oder Isopropanolammoniumsalz. Als Alkyl-$C_{10}$-$C_{16}$-ethersulfattensid kann ein Schwefelsäuremonoestersalz eines Anlagerungsproduktes von 1 bis 10 Mol Ethylenoxid an einen $C_{10}$-$C_{16}$-Fettalkohol eingesetzt werden.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Die Anwendung der erfindungsgemäßen Haarfärbemittel kann, unabhängig von der Art der kosmetischen Zubereitung, z. B. als Creme, Gel oder Shampoo, im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15 °C und 40 °C liegen.

Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo verwendet wurde.

## Beispiele

1. Herstellungsbeispiele

N-4-Aminophenyl-N'-2,4-diaminophenyl-ethylendiamin . 5 HCl . $H_2O$

1.1 N-4-Nitrophenyl-N'-2,4-dinitrophenyl-ethylendiamin

Eine Mischung aus 9,05 g (0,05 Mol) N-(p-Nitrophenyl)-ethylendiamin, 4,2 g (0,05 Mol) Natriumhydrogencarbonat und 100 ml Ethanol wurde auf 50 °C erwärmt. Bei dieser Temperatur wurden 9,3 g (0,05 Mol) 2,4-Dinitrofluorbenzol zugetropft. Nach 2 Stunden Sieden unter Rückfluß wurde abgekühlt und der Niederschlag abfiltriert. Es wurden gelbe Kristalle mit einem Schmelzpunkt von 242 °C erhalten.

1.2 N-4-Aminophenyl-N'-2,4-diaminophenyl-ethylendiamin . 5 HCl . $H_2O$

7 g des Reaktionsproduktes aus 1.1 wurde in 200 ml Ethanol gelöst und in Gegenwart von 0,5 g Palladium auf Kohle bei ca. 20 °C und 2 bar $H_2$-Druck hydriert. Nach beendeter Wasserstoff-Aufnahme wurde der Katalysator abfiltriert und das Filtrat mit Salzsäure angesäuert und zur Trockene eingedampft. Es wurden violette Kristalle mit einem Schmelzpunkt von 226 °C (unter Zersetzung) erhalten.

2. Anwendungsbeispiele

Es wurden erfindungsgemäße Haarfärbemittel in Form einer Haarfärbe-Cremeemulsion der folgenden Zusammensetzung hergestellt:

| | |
|---|---|
| Fettalkohol $C_{12-14}$ | 10,0 g |
| Fettalkohol $C_{12-14}$ + 2 EO-sulfat, Na-Salz, 28%ig | 25,0 g |
| Wasser | 60,0 g |
| N-4-Aminophenyl-N′-2,4-diaminophenyl-ethylendiamin . 5 HCl . $H_2O$ | 7,5 mMol |
| Kupplerkomponente | 7,5 mMol |
| $Na_2SO_3$ (Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3%iger Wasserstoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als Kuppler wurden die aus Tabelle I ersichtlichen Verbindungen eingesetzt. Dabei wurden die ebenfalls aus Tabelle I ersichtlichen Haaranfärbungen erhalten.

Die Färbungen gemäß Beispiel 2.1 bis 2.3 wurden zusätzlich in bezug auf ihre Lichtechtheit geprüft.

Prüfung der Lichtechtheit

Die Lichtechtheit der gefärbten Haarsträhnen wurde analog DIN 54004 (April 1966), Abschnitt 7.5.2 bestimmt. Die Methode besteht im wesentlichen darin, daß die gefärbten Haarsträhnen neben Textilproben, die mit 6 blauen, in ihrer Lichtechtheit abgestuften Typfärbungen des Lichtechtheitsmaßstabes mit einer Xenonbogenlampe mit einer Farbtemperatur von 5 500 bis 6 500 °K belichtet werden. Hierzu werden die Strähnchen und Textilproben nebeneinander auf einem Karton befestigt und die Randzonen der Strähnchen und Textilproben parallel zur Längskante des Probenträgers abgedeckt. Die Belichtung wird unter häufiger Kontrolle durch Abnehmen der Schablone solange durchgeführt, bis Typ 3 des Lichtechtheitsmaßstabes zwischen dem belichteten und dem unbelichteten Teil einen deutlich wahrnehmbaren Unterschied zeigt. Dann stellt man fest, ob die Proben Änderungen zeigen und bewertet diese gegebenenfalls im Vergleich zu den Änderungen der Typen 1, 2 und 3 des Lichtechtheitsmaßstabes. Dann wird weiter belichtet, bis Typ 4 des Lichtechtheitsmaßstabes zwischen dem nunmehr belichteten und dem unbelichteten Teil einen gerade wahrnehmbaren Farbunterschied zeigt. Dann tauscht man die Abdeckplatte durch eine größere aus, die etwa 1/2 der zuvor belichteten Fläche parallel zur Längskante abdeckt. Die Belichtung wird fortgesetzt, bis Typ 6 des Maßstabes einen eben wahrnehmbaren Farbunterschied zeigt. Die Bestimmung der Lichtechtheit erfolgt durch Vergleich der Kontraste auf den Haarsträhnchen mit den Kontrasten auf den Typfärbungen des Lichtechtheitsmaßstabes.

Die Ergebnisse der Lichtechtheitsprüfung sind ebenfalls der Tabelle I zu entnehmen.

Tabelle I

| Beispiel Nr. | Kuppler-Komponente | erhaltener Farbton | Lichtecht-heit |
|---|---|---|---|
| 2.1 | 1-Naphthol | dunkelrubin | 4 |
| 2.2 | Resorcin | braunoliv | 4 |
| 2.3 | m-Aminophenol | schwarz | 4 |
| 2.4 | 5-Amino-2-methylphenol | dunkelviolett | |
| 2.5 | 5-Amino-4-Chlor-2-methylphenol | schwarzviolett | |
| 2.6 | 2,4-Dichlor-3-aminophenol | schwarz | |
| 2.7 | 1,3-Bis-(2,4-diaminophenyl)-propan | blauschwarz | |

**Patentansprüche**

1. N-4-Aminophenyl-N'-2,4-diaminophenyl-alkylendiamine der Formel (I)

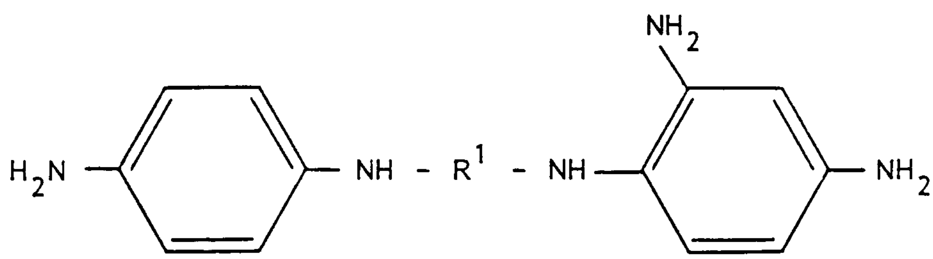

in der $R^1$ eine Gruppe $-C_nH_{2n}-$, worin n = 2 bis 4 ist oder eine Gruppe

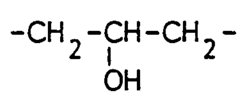

darstellt und deren wasserlösliche Salze.

2. N-4-Aminophenyl-N'-2,4-diaminophenyl-ethylendiamin

3. Verwendung der N-4-Aminophenyl-N'-2,4-diaminophenyl-alkylendiamine der Formel (I) gemäß den Ansprüchen 1 oder 2 oder deren Salze als Oxidationsfarbstoffvorprodukte vom Entwicklertyp in

Haarfärbemitteln.

**4.** Haarfärbemittel, enthaltend Oxidationsfarbstoffvorprodukte in einem kosmetischen Träger, dadurch gekennzeichnet, daß als Oxidationsfarbstoffvorprodukte N-4-Aminophenyl-N′-2,4-diaminophenylalkylendiamine der Formel (I) gemäß Anspruch 1 oder 2 oder deren wasserlösliche Salze als Entwicklerkomponente neben üblichen Entwickler- und Kupplerkomponenten und gegebenenfalls direktziehenden Haarfarbstoffen enthalten sind.

**5.** Haarfärbemittel gemäß Anspruch 4, dadurch gekennzeichnet, daß als Kupplerkomponenten Phenole, Naphthole, Resorcine und/oder 1,3-Bis-(2,4-diaminophenyl)-alkane oder 1,3-Bis-(2,4-diaminophenoxy)-alkane enthalten sind.

**6.** Haarfärbemittel gemäß Anspruch 4 oder 5, dadurch gekennzeichnet, daß Entwicklersubstanzen und Kupplersubstanzen im Molverhältnis 1 : 0,5 bis 1 : 2 enthalten sind, daß der Gehalt an Oxidationsfarbstoffvorprodukte 0,2 bis 5,0 Gew.-% des Haarfärbemittels und der Gehalt an N-4-Aminophenyl-N′-2,4-diaminophenylalkylendiaminen der Formel (I) oder deren Salzen 0,05 bis 10 Millimol pro 100 g des Haarfärbemittels beträgt.

**Claims**

**1.** N-4-aminophenyl-N'-2,4-diaminophenyl alkylenediamines corresponding to the following formula

(I) 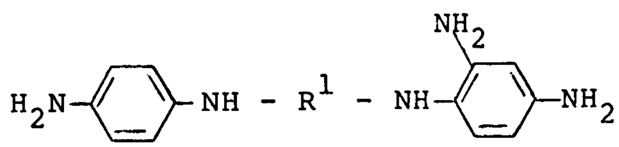

in which $R^1$ is a group of the formula $-C_nH_{2n}-$, where n = 2 to 4, or a group of the formula

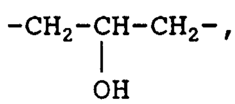

and water-soluble salts thereof.

**2.** N-4-aminophenyl-N'-2,4-diaminophenyl ethylenediamine.

**3.** The use of the N-4-aminophenyl-N'-2,4-diaminophenyl alkylenediamines corresponding to formula (I) claimed in claim 1 or 2 or salts thereof as oxidation dye precursors of the developer type in hair dyes.

**4.** Hair dyes containing oxidation dye precursors in a cosmetic carrier, characterized in that they contain as oxidation dye precursors the N-4-aminophenyl-N'-2,4-diaminophenyl alkylenediamines corresponding to formula (I) claimed in claim 1 or 2 or water-soluble salts thereof as developer component in addition to standard developer and coupler components and, optionally, substantive hair dyes.

**5.** Hair dyes as claimed in claim 4, characterized in that they contain phenols, naphthols, resorcinols and/or 1,3-bis-(2,4-diaminophenyl)-alkanes or 1,3-bis-(2,4-diaminophenoxy)-alkanes as coupler components.

**6.** Hair dyes as claimed in claim 4 or 5, characterized in that they contain developer substances and coupler substances in a molar ratio of 1:0.5 to 1:2 and in that they contain from 0.2 to 5.0% by weight, based on the hair dye, of oxidation dye precursors and from 0.05 to 10 millimols of N-4-aminophenyl-N'-2,4-diaminophenyl alkylenediamines corresponding to formula (I) or salts thereof per 100 g of the hair dye.

## Revendications

**1.** N-4-aminophényl-N'-2,4-diaminophényl-alkylènediamines de la formule (I)

(I) 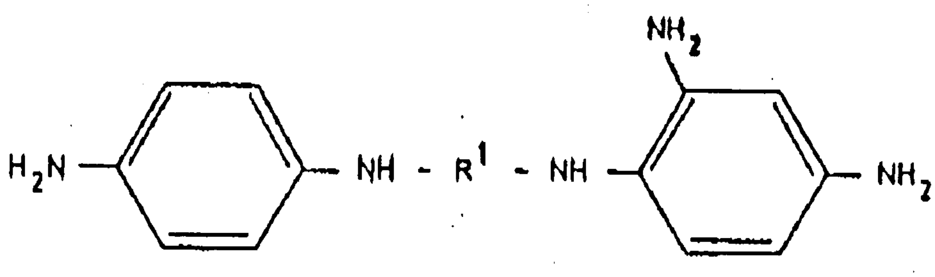

dans laquelle $R^1$ représente un groupe $-C_nH_{2n}-$, où n est un nombre de 2 à 4, ou un groupe

$$-CH_2-\underset{OH}{\underset{|}{C}}HCH_2-$$

et leurs sels solubles.

**2.** N-4-aminophényl-N'-2,4-diaminophényl-éthylènediamine.

**3.** Utilisation des N-4-aminophényl-N'-2,4-diaminophényl-alkylènediamines de la formule (I) selon la revendication 1 ou 2 ou de leurs sels comme précurseurs de colorants d'oxydation du type à développement dans les colorante pour cheveux.

**4.** Colorant pour cheveux contenant des précurseurs de colorants d'oxydation dans un support cosmétique, caractérisé en ce que les précurseurs de colorants d'oxydation sont des N-4-aminophényl-N'-2,4-diaminophényl-alkylènediamines de la formule (I) selon la revendication 1 ou 2 ou leurs sels solubles servant de constituants de développement, accompagnés des constituants usuels de développement et de couplage, et éventuellement de colorants pour cheveux agissant directement.

**5.** Colorant pour cheveux selon la revendication 4, caractérisé en ce qu'il contient comme constituants de couplage des phénols, des naphtols, des résorcines et/ou des 1,3-bis-(2,4-diaminophényl)-alcanes ou des 1,3-bis-(2,4-diaminophénoxy)-alcanes.

**6.** Colorant pour cheveux selon la revendication 4 ou 5, caractérisé en ce que les substances de développement et les substances de couplage sont présentes avec un rapport molaire allant de 1:0,5 à 1:2 et en ce que la teneur on précurseurs de colorants d'oxydation est comprise entre 0,2 et 5% en poids du colorant pour cheveux et que, la teneur en N-4-aminophényl-N'-2,4-diaminophényl-alkylènediamine de la formule (I) ou de leurs sels est comprise entre 0,05 et 10 millimoles pour 100 g de colorant pour cheveux.